# EUROPEAN PATENT SPECIFICATION

(11) **EP 0 878 202 B1**
(45) Date of publication and mention of the grant of the patent: **04.08.2004**
(21) Application number: 98108363.7
(22) Date of filing: 07.05.1998
(51) Int. Cl.: A61L 2/16, A61L 11/00, A61L 9/01

(54) **Product and method for sanitizing and deodorizing curbside bins for collecting waste in general**
Mittel und Verfahren zur Desinfektion und Desodorierung von städtischen Müllbehältern
Produit et procédé de désinfection et de désodorisation de poubelles urbaines

(30) Priority: 15.05.1997 IT MI971141
(43) Date of publication of application: 18.11.1998
(73) Proprietor: Eurovix S.r.l., 25046 Cazzago San Martino (Prov. of Brescia) (IT)
(72) Inventor: Bonassi, Dario, 25046 Cazzago San Martino (Brescia) (IT)
(74) Representative: Modiano, Guido, Dr.-Ing.

(56) References cited:
- FR-A- 2 658 077
- FR-A- 2 665 638
- US-A- 4 034 078

## Description

The present invention relates to a product and a method for sanitizing and deodorizing curbside bins for collecting waste in general.

Conventionally, in collecting municipal and non-municipal solid or liquid waste, after emptying the curbside bin or container by means of trucks known as trash trucks or trash compactor trucks, washing is periodically performed with specifically provided trucks, known as curbside bin washer trucks, using hot water or cold water or steam and sanitizing or disinfectant products of the chemical type, such as for example quaternary ammonium salts or products based on chlorine, formaldehyde or others.

After washing and drying, which is inevitably never perfect, the container or curbside bin of various sizes is deposited on the ground and is ready for use, i.e., to receive further waste.

Usually after a short period of time, due to the residual deposit of eluate and percolate, or of the parts of the waste that remain attached to the walls, or due to substances such as fungi, disease-causing bacteria and so forth, fermentation gases are released which produce unpleasant odors and cause discomfort in the environment and to people, both operators and users, since toxic gases, fungi, spores and disease-causing bacteria may be spread.

Moreover, the use of chemicals is effective for a short time, since the active principles contained in said chemicals lose their effectiveness one or two days after the treatment; accordingly, after this period the curbside bin usually resumes releasing unpleasant odors.

One of the aim of the present invention is to solve the above problem, by providing a product and a method for sanitizing and deodorizing curbside bins for collecting waste in general which can act both on the liquid material and on the solid material of the waste, achieving its neutralization for a very long period of time, which can vary between seven and twenty days, as a function of external weather conditions.

Within the scope of this aim, a particular object of the present invention is to provide a method which allows to sanitize and deodorize a curbside bin or container by using products in powder form which adhere to the walls of the container.

Another object of the present invention is to provide a product which can be applied very simply and quickly both manually and automatically, always achieving optimum results.

Another object of the present invention is to provide a product and a method which, by virtue of its particular characteristics of execution, is capable of giving the greatest assurances of reliability and safety in use and is also competitive from a merely economical point of view.

This aim, these objects and others which will become apparent hereinafter are achieved by a product for sanitizing and deodorizing curbside bins for collecting waste in general, according to the present invention, characterized in that it comprises a mix of enzyme powders and bacteria powders, preferably with mineral salts, a culture medium being further provided for the bacterial strains of said bacteria powders. Said enzyme and bacteria powders are advantageously provided in a percentage between 0.1 and 20% by weight.

Further characteristics and advantages of the present invention will become apparent from the following description of a preferred but not exclusive embodiment of a product and a method for sanitizing and deodorizing curbside bins for collecting waste in general.

The product for sanitizing and deodorizing curbside bins for collecting waste in general has the important particularity that it is constituted by a uniform mix of enzyme and bacteria powders which are distributed on the walls of the waste containers.

Said enzyme powders are preferably compositions containing at least one enzyme selected from the group comprising amylase, lipase, cellulase, protease, while bacteria powders comprise one or more bacterial strains, such as cocci and bacilli, with a suitable culture medium which can be constituted by agar-agar.

Said enzyme and bacteria powders are mixed with mineral salts in a percentage preferably between 0.1 and 20% by weight.

According to preferred embodiments, it is provided a powder with mixed particle size which comprises α-amylase enzymes, β-amylase enzymes, pentosanase enzymes, glucoamylase enzymes, cellulase enzymes, lactase enzymes, pancrease enzymes, protease enzymes, phosphorylase enzymes, hemicellulase enzymes, pectinase enzymes, β-glucanase enzymes, pullulanase enzymes and β-lactamase enzymes.

The bacteria are preferably constituted by micrococci and bacilli placed in a culture medium such as agar-agar.

The mineral salts are advantageously constituted by mineral salts of calcium and magnesium.

It is also possible to provide mordenite and dolomite, Lithothamnium calcareum algae, and active principles of Fucus and Laminaria.

The above-described composition is uniformly mixed and then packaged and prepared for manual or mechanical distribution on the walls of curbside bins or containers with an average sprinkling of 25 to 150 g as a function of the volume of the curbside bin.

As mentioned earlier, said spreading of the powders on the walls of the containers or curbside bins can be performed every seven to twenty days according to the user and to the environmental conditions, taking into account the fact that high temperatures increase the frequency of said treatments.

From the above description it is thus evident that the invention achieves the intended aim and objects; in particular, the fact is stressed that a product and a method for sanitizing and deodorizing curbside bins are provided which change the conventional criterion of using liquid products which are spread during washing and do not allow long-lasting action but are instead constituted by powders which, by adhering to the surfaces, determine a sanitizing process which is not chemical but enzyme- and bacteria-based, thus fully eliminating unpleasant odors.

## Claims

1. A product for sanitizing and deodorizing curbside bins for collecting waste in general, **characterized in that** it comprises a mix of enzyme powders and bacteria powders with mineral salts, a culture medium being further provided for the bacterial strains of said bacteria powders, said enzyme and bacteria powders being provided in a percentage between 0.1 and 20% by weight.

2. A sanitizing and deodorizing product according to claim 1, **characterized in that** said enzyme are selected from the group consisting of amylase, lipase, cellulase, protease and mixtures thereof.

3. A sanitizing and deodorizing product according to the preceding claims, **characterized in that** said enzymes are selected from the group consisting of alpha -amylase enzymes, beta-amylase enzymes, pentosanase enzymes, glucoamylase enzymes, cellulase enzymes, lactase enzymes, pancrease enzymes, protease enzymes, phosphorylase enzymes, hemicellulase enzymes, pectinase enzymes, beta -glucanase enzymes, pullulanase enzymes, beta -lactamase enzymes and mixtures thereof.

4. A sanitizing and deodorizing product according to one or more of the preceding claims, **characterized in that** said bacteria comprise at least one among the bacterial strains of micrococci and bacilli.

5. A sanitizing and deodorizing product according to one or more of the preceding claims, **characterized in that** said mineral salts comprise mineral salts of calcium and magnesium.

6. A sanitizing and deodorizing product according to one or more of the preceding claims, **characterized in that** said culture medium for bacterial strains is constituted by agar-agar.

7. A sanitizing and deodorizing product according to one or more of the preceding claims, **characterized in that** it comprises mordenite and dolomite.

8. A sanitizing and deodorizing product according to one or more of the preceding claims, **characterized in that** it comprises Lithothamnium calcareum algae.

9. A sanitizing and deodorizing product according to one or more of the preceding claims, **characterized in that** it comprises active principles of Fucus and Laminaria.

10. A method for sanitizing and deodorizing curbside bins for collecting waste in general, **characterized in that** it consists in spreading inside a curbside bin and the like a product according to claims 1 to 9.

11. A method according to claim 10, **characterized in that** said mix is sprinkled in an amount between 25 and 150 g per curbside bin.

## Revendications

1. Produit pour assainir et désodoriser des poubelles de trottoir pour la collecte d'ordures en général, **caractérisé en ce qu'**il comprend un mélange de poudres enzymatiques et de poudres bactériennes avec des sels minéraux, un milieu de culture étant en outre fourni pour les souches bactériennes desdites poudres bactériennes, lesdites poudres enzymatiques et bactériennes étant fournies en un pourcentage entre 0,1 et 20 % en poids.

2. Produit assainissant et désodorisant selon la revendication 1, **caractérisé en ce que** lesdites enzymes sont choisies dans le groupe constitué par une amylase, une lipase, une cellulase, une protéase et des mélanges de celles-ci.

3. Produit assainissant et désodorisant selon les revendications précédentes, **caractérisé en ce que** lesdites enzymes sont choisies dans le groupe constitué par les enzymes alpha-amylases, les enzymes bêta-amylases, les enzymes pentosanases, les enzymes glucoamylases, les enzymes cellulases, les enzymes lactases, les enzymes pancréases, les enzymes protéases, les enzymes phosphorylases, les enzymes hémicellulases, les enzymes pectinases, les enzymes bêta-glucanases, les enzymes pullulanases, les enzymes bêta-lactamases et des mélanges de celles-ci.

4. Produit assainissant et désodorisant selon une ou plusieurs des revendications précédentes, **caractérisé en ce que** lesdites bactéries comprennent au moins l'une parmi les souches bactériennes de microcoques et de bacilles.

5. Produit assainissant et désodorisant selon une ou plusieurs des revendications précédentes, **caractérisé en ce que** lesdits sels minéraux comprennent des sels minéraux de calcium et de magnésium.

6. Produit assainissant et désodorisant selon une ou plusieurs des revendications précédentes, **caractérisé en ce que** ledit milieu de culture pour souches bactériennes est constitué par de l'agar-agar.

7. Produit assainissant et désodorisant selon une ou plusieurs des revendications précédentes, **caractérisé en ce qu'**il comprend de la mordénite et de la dolomite.

8. Produit assainissant et désodorisant selon une ou plusieurs des revendications précédentes, **caractérisé en ce qu'**il comprend l'algue *Lithiothamnium calcareum*.

9. Produit assainissant et désodorisant selon une ou plusieurs des revendications précédentes, **caractérisé en ce qu'**il comprend les principes actifs de fucus et de laminaire.

10. Procédé pour assainir et désodoriser des poubelles de trottoir pour la collecte d'ordures en général, **caractérisé en ce qu'**il consiste à étaler à l'intérieur d'une poubelle de trottoir et similaire un produit selon les revendications 1 à 9.

11. Procédé selon la revendication 10, **caractérisé en ce que** ledit mélange est saupoudré en une quantité entre 25 et 150 g par poubelle de trottoir.

## Patentansprüche

1. Produkt zur Hygienisierung und Desodorierung von städtischen Abfallbehältern zum Sammeln von Müll im allgemeinen, **dadurch gekennzeichnet, daß** dieses aufweist eine Mischung von Enzympulvern und Bakterienpulvern mit Mineralsalzen, ein Kulturmedium, das weiterhin bereitgestellt wird für die Bakterienstämme der Bakterienpulver, wobei die Enzym- und Bakterienpulver bereitgestellt werden in einem prozentualen Bereich zwischen 0,1 und 20 Gew.-%.

2. Hygienisierungs- und Desodorierungsprodukt nach Anspruch 1, **dadurch gekennzeichnet, daß** die Enzyme ausgewählt sind aus der Gruppe, bestehend aus Amylase, Lipase, Cellulase, Protease und deren Mischungen.

3. Hygienisierungs- und Desodorierungsprodukt nach den vorhergehenden Ansprüchen, **dadurch gekennzeichnet, daß** die Enzyme ausgewählt sind aus der Gruppe, bestehend aus Alpha-Amylase-Enzymen, Beta-Amylase-Enzymen, Pentosanase-Enzymen, Glucoamylase-Enzymen, Cellulase-Enzymen, Lactase-Enzymen, Pankrease-Enzymen, Protease-Enzymen, Phosphorylase-Enzymen, Hemicellulase-Enzymen, Pektinase-Enzymen, Beta-Glucanase-Enzymen, Pullulanase-Enzymen, Beta-Lactamase-Enzymen und deren Mischungen.

4. Hygienisierungs- und Desodorierungsprodukt nach einem oder mehreren der vorhergehenden Ansprüche, **dadurch gekennzeichnet, daß** die Bakterien wenigstens einen unter den Bakterienstämmen von Micrococci und Bacilli beinhalten.

5. Hygienisierungs- und Desodorierungsprodukt nach einem oder mehreren der vorhergehenden Ansprüche, **dadurch gekennzeichnet, daß** die Mineralsalze Mineralsalze von Kalzium und Magnesium enthalten.

6. Hygienisierungs- und Desodorierungsprodukt nach einem oder mehreren der vorhergehenden Ansprüche, **dadurch gekennzeichnet, daß** das Kulturmedium für Bakterienstämme durch Agar-Agar aufgebaut ist.

7. Hygienisierungs- und Desodorierungsprodukt nach einem oder mehreren der vorhergehenden Ansprüche, **dadurch gekennzeichnet, daß** dieses Mordenit und Dolomit enthält.

8. Hygienisierungs- und Desodorierungsprodukt nach einem oder mehreren der vorhergehenden Ansprüche, **dadurch gekennzeichnet, daß** dieses Lithothamnium-Calcareum-Algae enthält.

9. Hygienisierungs- und Desodorierungsprodukt nach einem oder mehreren der vorhergehenden Ansprüche, **dadurch gekennzeichnet, daß** dieses aktive Grundbestandteile von Fucus und Laminaria enthält.

10. Verfahren zur Hygienisierung und Desodorierung von städtischen Abfallbehältern zum Sammeln von Müll im allgemeinen, **dadurch gekennzeichnet, daß** dieses aufweist das innenseitige Einsprühen eines städtischen Müllbehälters und ähnlichem mit einem Produkt nach einem der Ansprüche 1 bis 9.

11. Verfahren nach Anspruch 10, **dadurch gekennzeichnet, daß** die Mischung in einer Menge zwischen 25 und 150 g je städtischem Abfallbehälter ausgesprüht wird.
